# EUROPÄISCHE PATENTSCHRIFT

(11) **EP 0 541 189 B1**
(45) Veröffentlichungstag und Bekanntmachung des Hinweises auf die Patenterteilung: **18.12.1996**
(21) Anmeldenummer: 92250302.4
(22) Anmeldetag: 15.10.1992
(51) Int. Cl.: A61B 17/06

(54) **Verpackung mit Einzelfäden und Nadel-Faden-Kombinationen für chirurgische Zwecke**
Package with sutures and armed sutures
Emballage avec sutures et sutures ayant des aiguilles

(30) Priorität: 16.10.1991 DE 4134200
(43) Veröffentlichungstag der Anmeldung: 12.05.1993
(73) Patentinhaber: ETHICON INC., Somerville, New Jersey 08876-0151 (US)
(72) Erfinder: Brunken, Dieter, W2359 Hüttblek (DE)
(74) Vertreter: UEXKÜLL & STOLBERG

(56) Entgegenhaltungen:
- WO-A-89/07420
- FR-A- 2 391 735
- US-A- 3 162 307
- US-A- 4 034 850
- US-A- 4 413 727

## Beschreibung

Die Erfindung betrifft eine Verpackung mit Einzelfäden und Nadel-Faden-Kombinationen für chirurgische Zwecke, insbesondere als Innenverpackung für doppelt sterile Verpackungen.

Chirurgisches Nahtmaterial wird in Form von Einzelfäden in vorgeschnittenen Längen und in Form von mit Nadeln armierten Fäden, d.h. Nadel-Faden-Kombinationen, verwendet. Für die Fäden stehen verschiedene Materialarten, wie z.B. Seide, Polyamide, Polypropylen oder geflochtene Polyester, in unterschiedlichen Fadenstärken und Fadenlängen zur Verfügung. Für die Armierung der Fäden wird eine Vielzahl verschiedener gerader und gebogener Nadeln verwendet, die sich durch Nadelgröße, Nadelstärke und Schliffart unterscheiden.

Die Verpackung von chirurgischem Nahtmaterial hat eine sichere und sterile Aufbewahrung des Inhalts zu gewährleisten. Dabei soll eine schnelle und sichere Entnahme ohne Verwicklungen der einzelnen Fäden ermöglicht und darüber hinaus erreicht werden, daß die Fäden nach ihrer Entnahme nicht die Form, in der sie in der Verpackung lagen, wieder einnehmen (Fadenmemory).

Der Oberbegriff des Patentanspruchs 1 geht aus von einer Verpackung mit Nahtmaterial nach WO-A-8907420.

Aus WO-A-8907420 ist eine Verpackung mit Nadel-Faden-Kombinationen für chirurgische Zwecke bekannt. Auf einer Bodenplatte sind eine vordere und eine hintere Trägerleiste sowie eine dazwischenliegende Stützleiste angeordnet. Die Entnahmeteile mehrerer Nadel-Faden-Kombinationen sind in nach oben offene Führungseinschnitte der vorderen Trägerleiste eingesetzt, während die Endbereiche der Nadel-Faden-Kombinationen entsprechend von der hinteren Trägerleiste gehalten werden. An einer Längsseite ist die Bodenplatte mit einer Abdeckplatte verbunden, die auf die Bodenplatte umlegbar ist und diese dann bis zur vorderen Trägerleiste abdeckt. Die andere Längsseite der Abdeckplatte ist mit einer Trägerplatte für eine weitere Lage von Nadel-Faden-Kombinationen verbunden, welche im zusammengeklappten Zustand auf der Rückseite der Bodenplatte liegt.

Bislang war es üblich, chirurgisches Nahtmaterial in Form eines oder mehrerer, bezüglich der Materialart, Fadenstärke, Fadenlänge und des Nadeltyps gleichartiger Fäden oder Nadel-Faden-Kombinationen, in doppelt steriler Verpackung anzubieten, d.h. eingeschlossen in einer Primärverpackung und einer Sekundärverpackung. Vor oder während der Operation muß die zureichende, nicht steril arbeitende OP-Pflegekraft die Sekundärverpackung (overwrap) der jeweiligen Packung öffnen, damit die steril arbeitende OP-Pflegekraft die sterile Primärverpackung ergreifen kann und nach Öffnen der Primärverpackung die darin enthaltenen Einzelfäden bzw. Nadel-Faden-Kombinationen entnehmen und an den operierenden Arzt weiterreichen kann. Diese Verpackungsweise führt zu einer erheblichen Belastung des OP-Personals. Bei größeren Operationen, bei denen mehrere unterschiedliche Einzelfäden und Nadel-Faden-Kombinationen benötigt werden, ist eine derartige Einzelzureichung überaus zeitaufwendig.

Die Erfindung hat sich zur Aufgabe gestellt, eine Verpackung oder ein Verpackungssystem mit Einzelfäden und Nadel-Faden-Kombinationen für chirurgische Zwecke vorzuschlagen, die es ermöglicht, daß für eine bestimmte Operation sämtliche von einem Operateur individuell gewünschten und für die jeweilige Operation erforderlichen verschiedenen Nadel-Faden-Kombinationen und Einzelfäden in der Verpackung in einer doppelt sterilen Packung zur Verfügung stehen, so daß diese Verpackung nach Entfernen der Sekundärverpackung und der Primärverpackung mit der Gesamtheit der für die jeweilige Operation erforderlichen Einzelfäden und Nadel-Faden-Kombinationen der steril arbeitenden OP-Pflegekraft zur Verfügung steht, die dann die Einzelfäden und Nadel-Faden-Kombinationen dem Operateur zureicht.

Diese Aufgabe wird erfindungsgemäß gelöst durch eine Verpackung mit Nahtmaterial für chirurgische Zwecke, insbesondere als Innenverpackung für doppelt sterile Verpackungen, mit einer Bodenplatte mit einer im vorderen Bereich zwischen den Längskanten der Bodenplatte auf dieser befestigten vorderen Trägerleiste mit nach oben offenen Führungseinschnitten und einer am entgegengesetzten Ende zwischen den Längskanten der Bodenplatte auf dieser befestigten hinteren Trägerleiste mit nach oben offenen Führungseinschnitten, ein oder mehreren Stützleisten, die zwischen der vorderen und der hinteren Trägerleiste und parallel zu diesen auf der Bodenplatte befestigt sind, mehreren Nadel-Faden-Kombinationen gleicher oder unterschiedlicher Materialart, Fadenstärke, Fadenlänge und Nadeltyps, deren Entnahmeteil in den Führungseinschnitten der vorderen Führungsleiste gehalten ist und deren Endbereiche in hinteren Trägerleisten gehalten werden, einer Abdeckplatte, die materialeinheitlich mit der einen Längskante der Bodenplatte verbunden ist und in ihrer flächenmäßigen Ausdehnung die Bodenplatte abdeckend bis zur vorderen Trägerleiste auf diese umlegbar ist, wobei die Verpackung mit Nahtmaterial für chirurgische Zwecke dadurch gekennzeichnet ist, daß Einzelfäden gleicher oder unterschiedlicher Materialart, Fadenstärke und Fadenlänge vorgesehen sind, deren Entnahmeteil in den Führungseinschnitten der vorderen Führungsleiste gehalten ist und deren Endbereiche in hinteren Trägerleisten gehalten werden, daß auf der Abdeckplatte eine oder mehrere Stützleisten derartig befestigt sind, daß diese im Zusammenwirken mit den Stützleisten auf der Bodenplatte eine Fixierung der Nadel-Faden-Kombinationen und der Einzelfäden bewirken, daß eine Verschlußplatte vorgesehen ist, die materialeinheitlich mit der anderen Längskante der Bodenplatte verbunden ist und in ihrer flächenmäßigen Ausdehnung so bemessen ist, daß sie auf die die Bodenplatte abdeckende Abdeckplatte abdeckend auf diese umlegbar ist, und daß im vorderen Entnahmebereich die Bodenplatte über die Trägerleiste hinausreicht und an ihren beiden Längskanten jeweils eine materialmäßig mit diesen Bereichen der Bodenplatte verbundene Abdecklasche und Verschlußlasche aufweist, die wie Abdeckplatte und Verschlußplatte unabhängig von diesen übereinander klappbar sind.

Vorteilhafte Ausgestaltungen ergeben sich aus den Unteransprüchen.

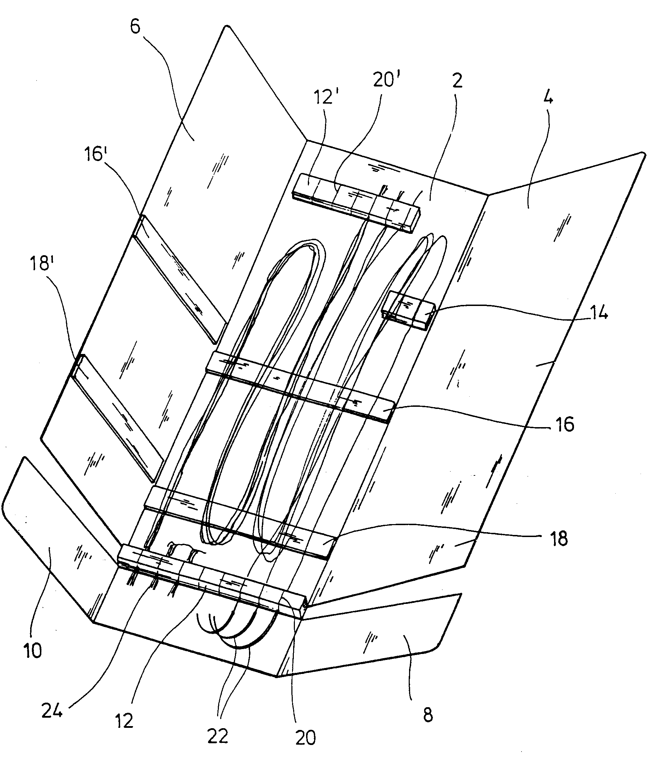

Die in der beiliegenden Figur gezeigte erfindungsgemäße Verpackung für Nadel-Faden-Kombinationen besteht aus einer Bodenplatte 2, die vorzugsweise rechteckig ist und ein Längsformat hat. Zwischen den Längskanten der Bodenplatte 2 ist im vorderen Entnahmebereich eine Trägerleiste 12 befestigt, die nach oben offene Führungseinschnitte 20 aufweist. Am entgegengesetzten Ende der Bodenplatte 2 ist zwischen deren Längskanten eine weitere hintere Trägerleiste 12' vorgesehen, die ebenfalls nach oben offene Führungseinschnitte 20' aufweist. In der vorderen Trägerleiste sind in den Einschnitten die verschieden dimensionierten armierten Nadeln 22 fixiert und ferner auch die Einzelfäden 24. Diese verlaufen schlingenförmig über die Bodenplatte 2 bis zur hinteren Trägerleiste 12', die mit ihrem nach oben offenen Führungseinschnitten 20' die Endteile der Fäden bzw. der mit den Nadeln armierten Fäden aufnimmt.

Die Ausbildung der Trägerleiste und insbesondere der vorderen Trägerleiste 12 kann den jeweiligen Anforderungen angepaßt werden.

Auf der Bodenplatte 2 sind ein oder mehrere Stützleisten 16 bzw. 18 aufgeklebt, die zwischen der vorderen und der hinteren Trägerleiste 12 bzw. 12' parallel zu diesen Stützleisten auf der Bodenplatte 2 befestigt, beispielsweise aufgeklebt, sind. Die Einzelfäden bzw. die mit Nadeln armierten Fäden sind schlingenartig auf dieser Bodenplatte bzw. auf diesen Stützleisten angeordnet. Auf der Abdeckplatte 6, die materialeinheitlich mit der einen Längskante der Bodenplatte 2 verbunden ist und die in ihrer flächenmäßigen Ausdehnung die Bodenplatte 2 im wesentlichen abdeckt, sind ein oder mehrere Stützleisten angeordnet, die mit den entsprechenden Stützleisten auf der Bodenplatte zusammenwirken oder eine Fixierung der jeweiligen Fäden ermöglichen. Im vorliegenden Ausführungsbeispiel sind auf der Bodenplatte 2 eine mittlere Stützleiste 16 und eine weitere vordere Stützleiste 18 vorgesehen, während auf der Abdeckplatte 6 eine weitere mittlere Stützleiste 16' und eine entsprechende Stützleiste 18' angeordnet sind. Diese Stützleisten sind so angeordnet, daß sie die Fäden und insbesondere die schlingenmäßig geführten Fäden in der Verpackung festhalten. Da es sich vorzugsweise um Kunststoffleisten handelt, die eine glatte Oberfläche haben, lassen sich die Fäden bzw. die mit Nadeln armierten Fäden ohne Schwierigkeiten aus den Einschnitten der Trägerleiste 12 herausziehen.

Letztlich ist eine materialmäßig mit der anderen Längskante der Bodenplatte 2 verbundene Verschlußplatte 4 vorgesehen, die über die die Bodenplatte 2 abdeckende Abdeckplatte 6 klappbar ist.

Der vordere Teil der Bodenplatte 2 besitzt noch eine materialmäßig an der Längskante angesetzte Abdecklasche 10 und Verschlußlasche 8, die ebenfalls flächenmäßig so ausgebildet sind, daß sie übereinanderklappbar sind.

Auf der Bodenplatte 2 können auch ein oder mehrere Führungsleisten 14 mit nach oben offenen Längsschnitten angeordnet sein, um die schlingenmäßig auf der Bodenplatte angeordneten Fäden ordnungsgemäß zum Entnahmebereich laufen zu lassen. Alle Trägerleisten, Führungsleisten oder Stützleisten bestehen vorzugsweise aus einem weichen porösen Kunststoff mit vorzugsweise glatter Oberfläche.

Die erfindungsgemäße Verpackung für Nadel-Faden-Kombinationen und Einzelfäden für chirurgische Zwecke wird in ihrer Gesamtheit mit den für den jeweiligen chirurgischen Eingriff erforderlichen und verschieden ausgebildeten Nadel-Fadenkombinationen in zugeklapptem Zustand in der gesonderten Primärverpackung, die sich noch in der Sekundärverpackung befindet, sterilisiert zur Verfügung gestellt. Die unter sterilen Bedingungen arbeitende OP-Pflegekraft braucht nach Öffnung der Sekundärverpackung durch die nicht steril arbeitende OP-Pflegekraft nur die Primärverpackung zu ergreifen und zu öffnen, die erfindungsgemäße Verpackung zu ergreifen und die Verschlußlasche 8 und die Abdecklasche aufzuklappen, wobei Abdeckplatte 6 und Verschlußplatte 4 geschlossen bleiben, so daß sie die Einzelfäden und Nadel-Faden-Kombinationen an den im vorderen Entnahmebereich zur Verfügung stehenden Fadenenden bzw. Nadeln ergreifen, entnehmen und dem Operateur zureichen kann.

## Patentansprüche

1. Verpackung mit Nahtmaterial für chirurgische Zwecke, insbesondere als Innenverpackung für doppelt sterile Verpackungen, mit
einer Bodenplatte (2) mit einer im vorderen Bereich zwischen den Längskanten der Bodenplatte (2) auf dieser befestigten vorderen Trägerleiste (12) mit nach oben offenen Führungseinschnitten (20) und einer am entgegengesetzten Ende zwischen den Längskanten der Bodenplatte (2) auf dieser befestigten hinteren Trägerleiste (12') mit nach oben offenen Führungseinschnitten (20'),
ein oder mehreren Stützleisten (16, 18), die zwischen der vorderen und der hinteren Trägerleiste (12, 12') und parallel zu diesen auf der Bodenplatte (2) befestigt sind,
mehreren Nadel-Faden-Kombinationen (22) gleicher oder unterschiedlicher Materialart, Fadenstärke, Fadenlänge und Nadeltyps, deren Entnahmeteil in den Führungseinschnitten (20) der vorderen Führungsleiste (12) gehalten ist und deren Endbereiche in hinteren Trägerleisten gehalten werden,
einer Abdeckplatte (6), die materialeinheitlich mit der einen Längskante der Bodenplatte (2) verbunden ist und in ihrer flächenmäßigen Ausdehnung die Bodenplatte (2) abdeckend bis zur vorderen Trägerleiste (12) auf diese umlegbar ist,
**dadurch gekennzeichnet,**
daß Einzelfäden (24) gleicher oder unterschiedlicher Materialart, Fadenstärke und Fadenlänge vorgesehen sind, deren Entnahmeteil in den Führungseinschnitten (20) der vorderen Führungsleiste (12) gehalten ist und deren Endbereiche in hinteren Trägerleisten gehalten werden,
daß auf der Abdeckplatte (6) eine oder mehrere Stützleisten (16', 18') derartig befestigt sind, daß diese im Zusammenwirken mit den Stützleisten (16, 18) auf der Bodenplatte eine Fixierung der Nadel-Faden-Kombinationen (22) und der Einzelfäden (24) bewirken,
daß eine Verschlußplatte (4) vorgesehen ist, die materialeinheitlich mit der anderen Längskante der Bodenplatte (2) verbunden ist und in ihrer flächenmäßigen Ausdehnung so bemessen ist, daß sie auf die die Bodenplatte (2) abdeckende Abdeckplatte (6) abdeckend auf diese umlegbar ist, und
daß im vorderen Entnahmebereich die Bodenplatte (2) über die Trägerleiste (12) hinausreicht und an ihren beiden Längskanten jeweils eine materialmäßig mit diesen Bereichen der Bodenplatte (2) verbundene Abdecklasche (10) und Verschlußlasche (8) aufweist, die wie Abdeckplatte und Verschlußplatte unabhängig von diesen übereinander klappbar sind.

2. Verpackung gemäß Anspruch 1, **dadurch gekennzeichnet**, daß auf der Bodenplatte (2) noch ein oder mehrere Führungsleisten (14) mit nach oben offenen Einschnitten angeordnet sind.

3. Verpackung nach einem der Ansprüche 1 und 2, **dadurch gekennzeichnet**, daß die Trägerleisten (12, 12'), die Führungsleisten (14) und Stützleisten (16, 16', 18, 18') aus Kunststoff und insbesondere aus Weich- oder Schaumkunststoff bestehen.

4. Verpackung nach Anspruch 1 bis 3, **dadurch gekennzeichnet**, daß die Nadel-Faden-Kombinationen (22) und die Einzelfäden (24) mehrmals über die Stützleisten (16, 18) schlingenartig gelegt sind und von den entsprechenden Stützleisten (16', 18') der Abdeckplatte (6) gehalten werden.

## Claims

1. Pack with suture material for surgical purposes, particularly as an inner pack for double sterile packs, comprising
a base plate (2) with a front carrier ledge (12) fixed thereto in the front region between the longitudinal edges of the base plate (2) and having upwardly open guide slits (20) and a rear carrier ledge (12') fixed to the base plate at the opposite end between the longitudinal edges of the base plate (2) and having upwardly open guide slits (20'),
one or more support ledges (16, 18), which are fixed between the front and the rear carrier ledge (12, 12') parallel thereto on the base plate (2),
several needle-thread combinations (22) with the same or different material type, thread thickness, thread length and needle type, whose removal part is kept in the guide slits (20) of the front carrier ledge (12) and whose end regions are held in rear carrier ledges,
a cover plate (6) which is connected in material-integral manner to one longitudinal edge of the base plate (2) and which can be wrapped thereover, covering the base plate (2) in its surface extension up to the front carrier ledge (12),
characterized
in that there are provided elementary threads (24) with the same or different material type, thread thickness and thread length, whose removal part is kept in the guide slits (20) of the front carrier ledge (12) and whose end regions are held in rear carrier ledges,
in that to the cover plate (6) there are fixed one or more support ledges (16', 18') in such a way that in cooperation with the support ledges (16, 18) on the base plate they bring about a fixing of the needle-thread combinations (22) and the elementary threads (24),
in that there is provided a closure plate (4), which is connected in material-integral manner to the other longitudinal edge of the base plate (2) and whose surface extension is dimensioned in such a way that it can be wrapped over the cover plate (6), covering the cover plate, which cover plate covers the base plate (2), and
in that in the front removal region the base plate (2) extends beyond the carrier ledge (12) and has on its two longitudinal edges a cover flap (10) and a closure flap (8) material-connected to said areas of the base plate (2) and which in the same way as the cover plate and the closure plate can be flapped over one another independently of the latter.

2. Pack according to Claim 1, characterized in that on the base plate (2) are additionally provided one or more guide ledges (14) with upwardly open slits.

3. Pack according to one of the Claims 1 and 2, characterized in that the carrier ledges (12, 12'), guide ledges (14) and support ledges (16, 16', 18, 18') are made from plastic and in particular soft or foam plastic.

4. Pack according to one of the Claims 1 to 3, characterized in that the needle-thread combinations (22) and the elementary threads (24) are looped several times over the support ledges (16, 18) and are secured by the corresponding support ledges (16', 18') of the cover plate (6).

## Revendications

1. Emballage de matériel de suture à usage chirurgical utilisé en particulier comme sous-emballage pour des emballages doublement stériles, comprenant un panneau de fond (2) présentant une barrette de support avant (12) fixée dans la zone avant sur le panneau de fond (2) entre les bords longitudinaux de celui-ci et pourvue d'incision de guidage (20) ouvertes vers le haut et une barrette de support arrière (12') fixée à l'extrémité opposée du panneau de fond (2) entre les bords longitudinaux de celui-ci et pourvue d'incision de guidage (20') ouvertes vers le haut, ainsi qu'une ou plusieurs barrettes d'appui (16, 18) qui sont fixées sur le panneau de fond (2) entre la barrette de support avant (12) et la barrette de support arrière (12') et parallèlement à celles-ci, plusieurs combinaisons aiguille-fil (22) de matière, de grosseur de fil, de longueur de fil et de type d'aiguille identiques ou différents, dont la partie de préhension est maintenue dans les incisions de guidage (20) de la barrette de guidage avant (12) et dont les parties d'extrémité sont maintenues dans les barrettes de support arrière, un panneau de recouvrement (6) relié d'un seul tenant à un des bords longitudinaux du panneau de fond (2) et pouvant être rabattu sur toute sa surface sur le panneau de fond (2) de manière à recouvrir celui-ci jusqu'à la barrette de support avant (12), caractérisé en ce que sont prévus des brins de fil sans aiguilles (24) de matière, de grosseur et de longueur identiques ou différentes dont la partie de préhension est maintenue dans les incisions de guidage (20) de la barrette de guidage avant (12) et dont les parties d'extrémité sont maintenues dans les barrettes de support arrière, en ce que sur le panneau de recouvrement (6) sont fixées une ou plusieurs barrettes d'appui (16', 18') de telle sorte que, en coopération avec les barrettes d'appui (16, 18) disposées sur le panneau de fond, elles assurent une fixation des combinaisons aiguille-fil (22) et des brins de fil sans aiguilles (24), en ce qu'un panneau de fermeture (4) est prévu, relié d'un seul tenant à l'autre bord longitudinal du panneau de fond (2) et dont la surface a une dimension telle qu'il peut être rabattu en position de recouvrement sur le panneau de recouvrement (6) recouvrant le panneau de fond (2), et en ce que, dans la zone de préhension avant, le panneau de fond (2) s'étend au-delà de la barrette de support (12) et présente, au niveau de chacun de ses deux bords longitudinaux, une patte de recouvrement (10) et une patte de fermeture (8) reliées d'un seul tenant à ces zones du panneau de fond (2) et peuvent être rabattues l'une sur l'autre comme le panneau de recouvrement et le panneau de fermeture, indépendamment de ceux-ci.

2. Emballage suivant la revendication 1, caractérisé en ce qu'encore une ou plusieurs barrettes de guidage (14) pourvues d'incisions ouvertes vers le haut sont disposées sur le panneau de fond (2).

3. Emballage suivant la revendication 1 ou la revendication 2, caractérisé en ce que les barrettes de support (12, 12'), la barrette de guidage (14) et les barrettes d'appui (16, 16', 18, 18') sont faites d'une matière plastique et en particulier d'une matière plastique souple ou d'une matière plastique cellulaire.

4. Emballage suivant l'une quelconque des revendications 1 à 3, caractérisé en ce que les combinaisons aiguille-fil (22) et les brins de fil sans aiguilles (24) sont disposés sur les barrettes d'appui (16, 18) en plusieurs boucles et sont maintenus par les barrettes d'appui correspondantes (16', 18') du panneau de recouvrement (6).
